# EUROPEAN PATENT SPECIFICATION

(11) **EP 0 511 289 B1**
(45) Date of publication and mention of the grant of the patent: **13.07.1994**
(21) Application number: 91903161.7
(22) Date of filing: 17.01.1991
(51) Int. Cl.: G01N 33/543, B01D 65/00, B01L 3/14

(54) **METHOD AND APPARATUS FOR TREATING FILTER MEMBRANES**
VERFAHREN UND VORRICHTUNG ZUR BEHANDLUNG VON FILTERMEMBRANEN
PROCEDE ET APPAREIL POUR TRAITER DES MEMBRANES DE FILTRAGE

(30) Priority: 17.01.1990 GB 9001081
(43) Date of publication of application: 04.11.1992
(73) Proprietor: ST. GEORGE'S ENTERPRISES LIMITED, London SW17 0RE (GB)
(72) Inventor: WINTERBOURNE, David, John Department of Surgery, Cranmer Terrace Tooting London SW17 0RE (GB)
(74) Representative: Marsden, John Christopher
(86) International application number: EP9100072
(87) International publication number: WO9110909

(56) References cited:
- EP-A- 0 052 862
- WO-A-20/0058
- DE-A- 3 635 966
- US-A-47 847 35

## Description

This invention relates to a method of treating filter membranes with liquid and apparatus of use therein, more particularly to a method and apparatus for uniformly treating filter membranes with small volumes of liquid.

It is frequently required to effect uniform treatment of a filter membrane with a liquid. Thus, for example, the need to bind ligands (e.g. radioactively labelled nucleic acid sequences complementary to a gene under investigation or antibodies specific to a target antigen) to molecules immobilised onto filter media is commonly encountered in molecular biology, e.g. in the detection and analysis of gene fragments using Southern or northern blotting techniques and in the analysis of antigens by immunoblotting.

It is vital in many such applications that the filter membrane comes into substantially completely uniform contact with the ligand binding reagent solution or other treatment liquid. Thus particular care must be taken to ensure that air bubbles are not present on the surface of the membrane since these may lead to the formation of untreated areas on the membrane with the consequent risk of potentially anomalous results.

A number of ligand binding devices which may be used to effect such treatment are available but are generally comparatively complex and expensive, involving the use of agitators, thermostatically-controlled ovens etc. and typically costing of the order of £1000.

Other cheaper methods include the use of plastic bags as reaction vessels; these, however, give rise to handling difficulties and lack of safety when, for example, handling radioactively labelled ligands.

It has now been found that uniform liquid treatment of a filter membrane can be accomplished in an efficient and economical manner in an extremely simple form of static apparatus, with low liquid volume requirements.

According to one aspect of the invention there is provided a method of uniformly treating a filter membrane with a liquid which comprises the steps of (i) positioning the membrane about the inner walls of a tubular reaction vessel containing a volume of said liquid; (ii) inserting into the vessel a cylindrical mandrel the length of which is less than the internal length of the vessel and the volume of which is such that it displaces the volume of liquid throughout a length of the vessel sufficient to cover the membrane when the vessel is upright; and (iii) sealing and shaking the vessel whereby movement of the mandrel generates a flow of liquid within the vessel and thereby ensures that any air bubbles are displaced from the membrane.

The method has widespread applications, for example in the preparation of treated filter membranes for use in a variety of assay techniques and in the further treatment of previously prepared filter membranes. It is especially useful in that only small volumes of liquid need be used, this being of considerable advantage where a reagent dissolved therein is scarce and/or expensive. In contrast to known methods, in which the filter membrane is treated in a horizontal position, the filter membrane may be positioned and held vertically during treatment so that air bubbles and/or any insoluble material are easily displaced therefrom.

The method is particularly useful in the treatment of filter membranes to effect binding of ligands to molecules immobilised onto the membrane. Thus according to a preferred aspect of the invention there is provided a method of binding a ligand to a molecule bound to a filter membrane which comprises the steps of (i) positioning the membrane carrying said molecule about the inner walls of a tubular reaction vessel containing a selected volume of reagent solution containing said ligand; (ii) inserting into the vessel a cylindrical mandrel the length of which is less (conveniently by up to 3%, e.g. by 1-2%) than the internal length of the vessel and the volume of which is such that it displaces the selected volume of reagent solution throughout a length of the vessel sufficient to cover the membrane when the vessel is upright; (iii) sealing and shaking the vessel whereby movement of the mandrel generates a flow of reagent solution within the vessel and thereby ensures that any air bubbles are displaced from the membrane; and (iv) incubating the vessel under appropriate conditions.

This method of the invention is applicable to all forms of ligand binding, for example between the following pairs of ligands: complementary nucleic acids; antigens and antibiodies; haptens and antibodies; sugars and lectins; enzymes and substrates; enzymes and inhibitors; biotin and avidin or streptavidin; and immunoglobulin and Protein A. Any of the above ligands may be linked covalently or otherwise to further molecules of interest and simply serve as means of immobilising the latter. In particular, one of the ligand binding partners may carry a label, for example a radionuclide, an enzyme, a fluorescent label or a dye.

This method of the invention may be used to locate a substrate bound to a filter membrane in more than one stage, a first ligand being bound initially to the substrate followed by a labelled ligand binding to the first ligand.

This method of the invention is thus of use in all forms of assay based on binding of ligands, which may loosely be termed immunoassays, e.g. competitive binding and sandwich assays using any of the labels described above.

The filter membrane may for example be cellulose nitrate paper, nylon or chemically reactive paper as conventionally used in northern blotting. Advantageously the membrane is positioned in the vessel by being pre-wetted and then wrapped around the mandrel; the mandrel may then be used to insert the membrane into the vessel, whereupon the mandrel is rotated to loosen and partially unwind the membrane. The mandrel may then advantageously be removed to ensure release of the membrane, whereafter the mandrel may be fully inserted into the vessel, displacing reagent solution to cover the membrane. The vessel may then be sealed, conveniently by means of a screw cap.

The volume of the reagent solution used is advantageously such that the solution substantially completely fills the vessel when the mandrel is fully inserted, thereby minimising the volume of air present when the vessel is sealed. The respective diameters of the mandrel and the inside of the vessel may thus be selected so that a low volume of solution is required, the principal limitation being the need to allow a tolerance so that the filter membrane can be accomodated.

Ligand binding in accordance with the preferred method of the invention may be carried out in more than one stage, without removal of the membrane from the vessel. Thus, for example, in the case of hybridization the membrane may initially be treated and incubated with pre-hybridization buffer and subsequently with an appropriately labelled probe/hybridization buffer solution.

Incubation is conveniently effected by standing the vessel in a water bath at the appropriate temperature. It is not necessary to agitate the vessel since the membrane is completely immersed in the reagent solution so allowing free diffusion of the ligand or other reagent. It may, however, be advantageous to repeat the shaking step at least once during incubation to ensure displacement of any further air bubbles from the membrane and further to ensure freedom of the membrane from the mandrel.

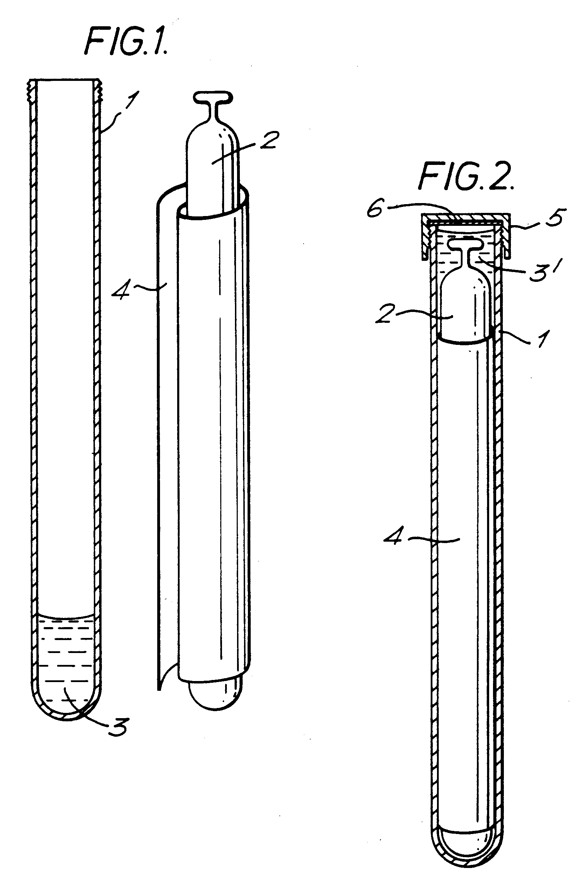

In the accompanying drawings, which serve to illustrate a preferred method of the invention without in any way limiting it:-
Fig. 1 represents a reaction vessel 1 and mandrel 2 as arranged at the start of a hybridization process, and
Fig. 2 represents the vessel 1 and mandrel 2 during an incubation stage.

At the commencement of a hybridization according to a preferred embodiment of the invention, an aliquot of pre-hybridization buffer 3 is pipetted into vessel 1; conveniently the relative sizes of vessel 1 and mandrel 2 are such that aliquots as small as 10ml may be used to treat a membrane of 300 cm² area.

The filter membrane 4 carrying a bound first ligand reactive with a labelled second ligand is pre-wetted and wound about mandrel 2, which is then inserted in vessel 1 and twisted to loosen and partly unwind the membrane 4. The mandrel 2 tends to float in the buffer 3, facilitating such manipulative steps, and may at this stage optionally be fully withdrawn and then reinserted to ensure release of the membrane 4, this additionally tending to prevent non-specific binding of material, with consequent reduction in background. The mandrel 2 is then gently pressed fully into the vessel 1, displacing buffer 3′ to cover the membrane 4, and cap 5 is screwed on to seal the vessel 1 by means of internal, optionally integral seal 6.

Shaking of the vessel 1 causes longitudinal movement of the mandrel 2, which has a pumping action on the buffer 3′. This assists in positioning the membrane 4 towards the bottom of the vessel 1 and displacing any air bubbles therefrom. The pre-hybridization stage is then completed by standing the vessel in, for example, an incubator or a water bath at the requisite temperature.

Cap 5 and mandrel 2 are then removed and all or part of the pre-hybridization buffer is withdrawn, e.g. by pipette, to be replaced by an equivalent volume of probe/hybridization buffer containing the appropriately labelled second ligand. Mandrel 2 is then carefully reinserted, slowly displacing the buffer/probe over the membrane 4, and cap 5 is replaced and secured.

The vessel 1 is again shaken to ensure that air bubbles are displaced from the membrane 4, and is then subjected to appropriate incubation conditions. It may be advisable to check for formation of further bubbles on the membrane 4 after about 10 minutes of incubation and, if necessary to shake the vessel 1 again to displace these.

Upon completion of hybridization, cap 5 and mandrel 2 are removed; where a radioactive probe has been employed it is advantageous to conduct this operation over a sink, washing the mandrel 2 directly under running water. The hybridization buffer/probe solution may then be removed from vessel 1 by means of a pipette and disposed of or transferred to storage. The membrane 4 may then be washed in situ in the vessel 1, advantageously without the mandrel 2 being present; the vessel 1 may thus be completely filled, thereby permitting the use of maximum volumes of wash. A succession of treatments with low stringency wash buffer (e.g. in amounts of about 50 ml) may be made in this way to remove unbound probe material, as may further higher stringency washings.

Alternatively the high stringency buffer washing may take place in a separate vessel (e.g. a plastic box) after removal of the membrane 4 from vessel 1, e.g. with the aid of forceps; this latter procedure is advantageous where, for example, it is desired to monitor a ³²P probe with a Geiger counter. In general, however, it is preferred to conduct as many process steps as possible while the membrane 4 is contained in the vessel 1, this being particularly advantageous for safety reasons where radioactive materials are being handled. Thus, for example, the membrane 4 may be stripped in situ in the vessel 1, e.g. by boiling, to remove a particular probe and then retested by treatment with a further probe.

It will be appreciated that the hybridization method specifically described above is equally applicable to other forms of ligand binding.

According to a further aspect of the invention there is provided apparatus of use in the method of the invention, said apparatus comprising a tubular vessel having associated therewith a cylindrical mandrel the length of which is up to 3% (e.g. 1-2%) less than the internal length of the vessel and the cross-sectional area of which is up to 30% (e.g.10-25%) less than the internal cross-sectional area of the vessel, said vessel also possessing closure means, e.g. a screw top.

The vessel and mandrel are conveniently made of glass, although various plastics materials may also be used provided they possess nonadsorbent surfaces inert to the intended reagents and can withstand the intended operating temperatures (e.g. up to 100°C). Suitable plastics materials thus include perspex and polypropylene.

The tubular vessel may, for example, have a volume in the range 10-500 ml, the use of vessels having a volume of about 50 ml and a length of about 20 cm having proved convenient in practice.

The closure means may conveniently comprise a screw cap made from a rigid plastics material and adapted to fit a screw thread on the exterior wall of the tubular vessel. Such a cap is advantageously fitted with a rubber liner coated with polytetrafluoroethylene or similar inert material on the surface facing the vessel, to ensure formation of a substantially absolute and inert seal with the vessel. Alternatively the cap may be formed from a more pliable material, e.g. a soft plastics material such as polypropylene, and provided with an integral internal seal, e.g. by an appropriate angled configuration.

The lower end of the mandrel and the interior of the closed end of the vessel are desirably complementary in shape to ensure a close fit; both may, for example, advantageously be rounded.

If desired for use in treatment involving radioactive ligands, the vessel may additionally incorporate a shield, for example a thick perspex coating or a fillable water jacket. The shield may optionally be designed to render the vessel free-standing.

According to a still further aspect of the invention there are provided kits for use in the method of the invention which comprise apparatus in accordance with the invention and one or more filter membranes carrying ligand-binding molecules attached thereto, optionally together with one or more reagents for use in an assay.

## Claims

1. A method of uniformly treating a filter membrane (4) with a liquid (3) which comprises the steps of (i) positioning the membrane (4) about the inner walls of a tubular reaction vessel (1) containing a volume of said liquid (3); (ii) inserting into the vessel (1) a cylindrical mandrel (2) the length of which is less than the internal length of the vessel (1) and the volume of which is such that it displaces the volume of liquid (3) throughout a length of the vessel (1) sufficient to cover the membrane (4) when the vessel (1) is upright; and (iii) sealing and shaking the vessel (1) whereby movement of the mandrel (2) generates a flow of liquid (3) within the vessel and thereby ensures that any air bubbles are displaced from the membrane (4).

2. A method of binding a ligand to a molecule bound to a filter membrane (4) which comprises the steps of (i) positioning the membrane (4) carrying said molecule about the inner walls of a tubular reaction vessel (1) containing a selected volume of reagent solution (3) containing said ligand; (ii) inserting into the vessel a cylindrical mandrel (2) the length of which is less than the internal length of the vessel (1) and the volume of which is such that it displaces the selected volume (3) of reagent solution throughout a length of the vessel (1) sufficient to cover the membrane (4) when the vessel (1) is upright; (iii) sealing and shaking the vessel (1) whereby movement of the mandrel (2) generates a flow of reagent solution (3) within the vessel (1) and thereby ensures that any air bubbles are displaced from the membrane (4); and (iv) incubating the vessel (1) under appropriate conditions.

3. A method as claimed in claim 2 wherein ligand binding is effected between any of the following pairs of ligands: complementary nucleic acids; antigens and antibodies; haptens and antibodies; sugars and lectins; enzymes and substrates; enzymes and inhibitors; biotin and avidin or streptavidin; and immunoglobin and Protein A.

4. A method as claimed in claim 3 wherein one of the ligands carries a label.

5. A method as claimed in claim 4 wherein the label is a radionuclide, an enzyme, a fluorescent label or a dye,

6. A method as claimed in any of the preceding claims wherein the length of the mandrel (2) is 1-2% less than the internal length of the vessel (1).

7. A method as claimed in any of the preceding claims wherein the filter membrane (4) is cellulose nitrate paper, nylon or chemically reactive paper.

8. A method as claimed in any of the preceding claims wherein the membrane (4) is positioned about the inner walls of the vessel (1) by prewetting and wrapping the membrane (4) around the mandrel (2), inserting the mandrel (2) into the vessel (1) and rotating the mandrel (2) to loosen and partially unwind the membrane (4).

9. A method as claimed in claim 8 wherein the mandrel (2) is removed and reinserted after rotation to loosen and partially unwind the membrane (4).

10. A method as claimed in any of the preceding claims wherein the volume of ligand or reagent solution (3) is such that the ligand or solution (3) substantially completely fills the vessel (1) when the mandrel (2) is fully inserted therein.

11. A method as claimed in any of the preceding claims wherein a plurality of treatments are effected without removing the membrane (4) from the vessel (1).

12. A method as claimed in claim 11 comprising successive treatments with pre-hydridization buffer, a labelled probe/hybridization buffer solution and wash solutions.

13. Apparatus for use in a method as claimed in claim 1 comprising a tubular vessel (1) having associated therewith a cylindrical mandrel (2) the length of which is up to 3% less than the internal length of the vessel (1) and the cross-sectional area of which is up to 30% less than the internal cross-sectional area of the vessel (1), said vessel also possessing closure means.

14. Apparatus as claimed in claim 13 wherein the closure means comprise a screw cap (5).

15. Apparatus as claimed in claim 13 or claim 14 wherein the lower end of the mandrel (2) and the interior of the closed end of the vessel (1) are of complementary shape.

16. Apparatus as claimed in any of claims 13 to 15 further incorporating a shield.

17. Apparatus as claimed in claim 16 in which the shield renders the vessel (1) free-standing.

18. A kit for use in a method as claimed in claim 1 comprising an apparatus as claimed in claim 13 and one or more filter membranes carrying ligand-binding molecules attached thereto.

19. A kit as claimed in claim 18 also comprising one or more reagents for use in an assay.

## Patentansprüche

1. Verfahren zum gleichmäßigen Behandeln einer Filtermembran (4) mit einer Flüssigkeit (3), mit den folgenden Schritten: (i) Positionieren der Membran (4) entlang der Innenwand eines rohrförmigen Reaktionsbehälters (1), der ein Volumen der Flüssigkeit (3) enthält; (ii) Einsetzen, in den Behälter (1), eines zylindrischen Dorns (2), dessen Länge kleiner als die Innenlänge des Behälters (1) ist, und dessen Volumen dergestalt ist, daß er das Volumen der Flüssigkeit (3) über diejenige ganze Länge des Behälters (1) verdrängt, die dafür ausreicht, die Membran (4) zu überdecken, wenn der Behälter (1) aufrecht steht; und (iii) Abdichten und Schütteln des Behälters (1), wobei die Bewegung des Dorns (2) eine Strömung der Flüssigkeit (3) innerhalb des Behälters erzeugt und dadurch gewährleistet, daß alle Luftblasen von der Membran (4) verdrängt werden.

2. Verfahren zum Binden eines Liganden an ein Molekül, das an eine Filtermembran (4) gebunden ist, das die folgenden Schritte aufweist: (i) Positionieren der das Molekül tragenden Membran (4) entlang der Innenwand eines rohrförmigen Reaktionsbehälters (1), der ein ausgewähltes Volumen einer den Liganden enthaltenden Reagenzlösung (3) enthält; (ii) Einsetzen, in den Behälter, eines zylindrischen Dorns (2), dessen Länge kleiner als die Innenlänge des Behälters (1) ist, und dessen Volumen dergestalt ist, daß er das ausgewählte Volumen (3) der Reagenzlösung über eine Länge des Behälters (1) verdrängt, die dazu ausreicht, die Membran (4) zu überdecken, wenn der Behälter (1) aufrecht steht; (iii) Abdichten und Schütteln des Behälters (1), wodurch die Bewegung des Dorns (2) eine Strömung der Reagenzlösung (3) innerhalb des Behälters (1) erzeugt, wodurch gewährleistet ist, daß alle Luftblasen von der Membran (4) verdrängt werden; und (iv) Bebrüten des Behälters (1) unter geeigneten Bedingungen.

3. Verfahren nach Anspruch 2, bei dem die Ligandenbindung zwischen einem der folgenden Ligandenpaare hervorgerufen wird: komplementäre Nukleinsäuren; Antigene und Antikörper; Haptene und Antikörper; Zucker und Lektine, Enzyme und Substrate; Enzyme und Inhibitoren; Biotin und Avidin oder Streptavidin; und Immunglobulin und Protein A.

4. Verfahren nach Anspruch 3, bei dem einer der Liganden eine Markierung trägt.

5. Verfahren nach Anspruch 4, bei dem die Markierung ein Radionuklid, ein Enzym, eine Fluoreszenzmarkierung oder ein Farbstoff ist.

6. Verfahren nach einem der vorstehenden Ansprüche, bei dem die Länge des Dorns (2) 1 - 2 % kleiner als die Innenlänge des Behälters (1) ist.

7. Verfahren nach einem der vorstehenden Ansprüche, bei dem die Filtermembran (4) Zellulosenitrat-Papier, Nylon oder chemisch reagierendes Papier ist.

8. Verfahren nach einem der vorstehenden Ansprüche, bei dem die Membran (4) dadurch entlang der Innenwand des Behälters (1) angeordnet wird, daß diese Membran vorbefeuchtet und um den Dorn (2) gewickelt wird, der Dorn (2) in den Behälter (1) eingeführt wird und der Dorn (2) verdreht wird, um die Membran (4) zu lockern und teilweise abzuwickeln.

9. Verfahren nach Anspruch 8, bei dem der Dorn (2) nach der Drehung zum Lockern und teilweise Abwickeln der Membran (4) entfernt und wieder eingeführt wird.

10. Verfahren nach einem der vorstehenden Ansprüche, bei dem das Volumen des Liganden oder der Reagenzlösung (3) dergestalt ist, daß der Ligand oder die Lösung (3) den Behälter (1) im wesentlichen ganz auffüllt, wenn der Dorn (2) ganz in ihn eingeführt ist.

11. Verfahren nach einem der vorstehenden Ansprüche, bei dem mehrere Behandlungen ausgeführt werden, ohne daß die Membran (4) dem Behälter (1) entnommen wird.

12. Verfahren nach Anspruch 11, mit aufeinanderfolgenden Behandlungen mit einem Vorbastardisierungspuffer, einer markierten Sonde/Bastardisierung-Pufferlösung und Waschlösungen.

13. Vorrichtung zur Verwendung bei einem Verfahren gemäß Anspruch 1, mit einem rohrförmigen Behälter (1), dem ein zylindrischer Dorn (2) zugeordnet ist, dessen Länge bis zu 3 % kleiner als die Innenlänge des Behälters (1) ist und dessen Querschnittsfläche bis zu 30 % kleiner als die Innenquerschnittsfläche des Behälters (1) ist, wobei der Behälter auch über eine Verschlußeinrichtung verfügt.

14. Vorrichtung nach Anspruch 13, bei dem die Verschlußeinrichtung eine Schraubkappe (5) aufweist.

15. Vorrichtung nach Anspruch 13 oder Anspruch 14, bei der das untere Ende des Dorns (2) und der Innenraum des geschlossenen Endes des Behälters (1) zueinander komplementäre Formen aufweisen.

16. Vorrichtung nach einem der Ansprüche 13 bis 15, mit ferner einer Abschirmung.

17. Vorrichtung nach Anspruch 16, bei der die Abschirmung den Behälter (1) frei stehend macht.

18. Bausatz zur Verwendung bei einem Verfahren nach Anspruch 1, mit einer Vorrichtung nach Anspruch 13 und einer Filtermembran oder mehreren, die an ihnen angebrachte Liganden bindende Moleküle tragen.

19. Bausatz nach Anspruch 18 mit ferner einem oder mehreren Reagenzien zu Verwendung in einem Test.

## Revendications

1. Procédé de traitement uniforme d'une membrane de filtrage (4) avec un liquide (3) qui comprend les étapes consistant à (i) placer la membrane (4) sur les parois intérieures d'un récipient tubulaire de réaction (1) contenant un volume dudit liquide (3) ; (ii) insérer dans le récipient (1) un mandrin cylindrique (2) dont la longueur est inférieure à la longueur intérieure du récipient (1) et dont le volume est tel qu'il déplace le volume de liquide (3) sur une longueur du récipient (1) suffisante pour recouvrir la membrane (4) quand le récipient est droit ; et (iii) fermer le récipient (1) et le secouer de sorte que le mouvement du mandrin (2) engendre un courant de liquide (3) au sein du récipient et assure ainsi que toutes le bulles d'air sont enlevées de la membrane (4).

2. Procédé de liaison d'un ligand à une molécule liée à une membrane de filtrage (4) qui comprend les étapes consistant à (i) placer la membrane (4) portant ladite molécule sur les parois intérieures d'un récipient tubulaire de réaction (1) contenant un volume choisi de solution réactive (3) contenant ledit ligand ; (ii) insérer dans le récipient (1) un mandrin cylindrique (2) dont la longueur est inférieure à la longueur intérieure du récipient (1) et dont le volume est tel qu'il déplace le volume choisi de solution réactive (3) sur une longueur du récipient (1) suffisante pour recouvrir la membrane (4) quand le récipient est droit ; et (iii) fermer le récipient (1) et le secouer de sorte que le mouvement du mandrin (2) engendre un courant de solution réactive (3) au sein du récipient (1) et assure ainsi que toutes le bulles d'air sont enlevées de la membrane (4) ; et (iv) incuber le récipient (1) dans des conditions appropriées.

3. Procédé selon la revendication 2 dans lequel la liaison du ligand s'effectue entre l'une quelconque des paires suivantes de ligands : acides nucléiques complémentaires ; antigènes et anticorps ; haptènes et anticorps ; sucres et lectines ; enzymes et substrats ; enzymes et inhibiteurs ; biotine et avidine ou streptavidine ; et immunoglobine et protéine A.

4. Procédé selon la revendication 3 dans lequel l'un des ligands porte un marqueur.

5. Procédé selon la revendication 4 dans lequel le marqueur est un radionucléide, une enzyme, un marqueur fluorescent ou une teinture.

6. Procédé selon l'une quelconque des revendications précédentes dans lequel la longueur de mandrin (2) est de 1 à 2% inférieure à la longueur intérieure du récipient (1).

7. Procédé selon l'une quelconque des revendications précédentes dans lequel la membrane de filtrage (4) est du papier de nitrate de cellulose, du nylon ou un papier réactif chimique.

8. Procédé selon l'une quelconque des revendications précédentes dans lequel la membrane (4) est placée sur les parois intérieures du récipient (1) en préhumidifiant et enveloppant la membrane (4) autour du mandrin (2), en insérant le mandrin (2) dans le récipient (1) et en faisant tourner le mandrin (2) pour relâcher et dérouler partiellement la membrane (4).

9. Procédé selon la revendication 8 dans lequel le mandrin (2) est retiré et réinséré après rotation pour relâcher et partiellement dérouler la membrane.

10. Procédé selon l'une quelconque des revendications précédentes dans lequel le volume de ligand ou de solution réactive (3) est tel que le ligand ou la solution (3) remplit sensiblement totalement le récipient (1) quand le mandrin (2) y est complètement inséré.

11. Procédé selon l'une quelconque des revendications précédentes dans lequel on effectue plusieurs traitements sans retirer la membrane (4) du récipient (1).

12. Procédé selon la revendication 11 comprenant des traitements successifs avec un tampon de pré-hybridrisation, une solution échantillon marqué / tampon d'hybridisation et des solutions de lavage.

13. Appareil pour la mise en oeuvre du procédé selon la revendication 1 comprenant un récipient tubulaire (1) auquel est associé un mandrin cylindrique (2) dont la longueur est jusqu'à 3% inférieure à la longueur intérieure du récipient (1) et la section transversale est jusqu'à 30% inférieure à la section transversale intérieure du récipient (1), ledit récipient comprenant aussi des moyens de fermeture.

14. Appareil selon la revendication 13 dans lequel les moyens de fermeture comprennent un couvercle vissable (5).

15. Appareil selon la revendication 13 ou 14 dans lequel l'extrémité inférieure du mandrin (2) et l'intérieur de l'extrémité fermée du récipient (1) sont de forme complémentaire.

16. Appareil selon l'une quelconque des revendications 13 à 15 comprenant en outre un blindage.

17. Appareil selon la revendication 16 dans lequel le blindage rend le récipient (1) auto-stable.

18. Ensemble destiné à être utilisé dans un procédé selon la revendication 1 comprenant un appareil comme revendiqué dans la revendication 13 et une ou plusieurs membranes de filtrage auxquelles sont attachées des molécules de liaison de ligand.

19. Ensemble selon la revendication 18 comprenant également un ou plusiers réactifs utilisables dans une analyse.
